# FASCICULE DE BREVET EUROPEEN

(11) **EP 0 623 019 B1**
(45) Date de publication et mention de la délivrance du brevet: **10.05.2000**
(21) Numéro de dépôt: 94900873.4
(22) Date de dépôt: 24.11.1993
(51) Int. Cl.: A61K 31/23

(54) **MEDICAMENTS A BASE D'ACIDES DOCOSAHEXAENOIQUE COMME ANTIAGREGANTS PLAQUETTAIRES ET CONTRE DES CARENCES CEREBRALES EN ACIDES GRAS ESSENTIELS ET DES PROCEDES DE PREPARATION.**
Medikamente auf der Basis von Docosahexaensäure, als Plättchenaggregationshemmer und gegen cerebralen Mangel an Fettsäuren, und Verfahren zur Herstellung.
DOCOSAHEXAENOIC ACID BASED DRUGS, FOR USE AS PLATELET AGGREGATION INHIBITORS AND AGAINST ESSENTIAL FATTY ACID DEFICIENCIES OF THE BRAIN, AND PREPARATION PROCESSES.

(30) Priorité: 24.11.1992 FR 9214078
(43) Date de publication de la demande: 09.11.1994
(73) Titulaire: INSTITUT NATIONAL DE LA SANTE ET DE LA RECHERCHE MEDICALE (INSERM), 75654 Paris Cédex 13 (FR); IMEDEX, F-69630 Chaponost (FR)
(72) Inventeur: BAYON, Yves, F-69200 Vénissieux (FR); CROSET, Martine, F-69500 Bron (FR); LAGARDE, Michel, F-69680 Chassieu (FR); LECERF, Jean, F-21110 Genlis (FR); THIES, Frank, F-21000 Dijon (FR); TAYOT, Jean-Louis, F-69890 La-Tour-de-Salvagny (FR); CHIROUZE, Véronique, F-69110 Sainte-Foy-les-Lyon (FR)
(74) Mandataire: Pernez, Helga
(86) Numéro de dépôt international: FR9301158
(87) Numéro de publication internationale: WO9412170

(56) Documents cités:
- EP-A- 0 304 603
- EP-A- 0 342 795
- DE-A- 3 213 744
- GB-A- 2 218 904
- CHEMICAL ABSTRACTS, vol. 112, no. 10, 5 Mars 1990, Columbus, Ohio, US; abstract no. 84150r, NISHIZAWA Y. ET AL. 'Docosahexaenoic acid-containing phosphatidylcholines' page 444 ;colonne 1 ; cité dans la demande & JP,A,0 150 890 (...) 27 Février 1989
- CHEMICAL ABSTRACTS, vol. 116, no. 9, 2 Mars 1992, Columbus, Ohio, US; abstract no. 76383m, HIBINO H. ET AL 'Pharmaceuticals containing sn-1-oleoyl-sn-2 docosahexaenoylglycerophosphocholine for promoting and increasing sleep.' page 87 ; & JP,A,03 178 931 (...) 2 Aoüt 1991
- CHEMICAL ABSTRACTS, vol. 112, no. 15, 9 Avril 1990, Columbus, Ohio, US; abstract no. 132469a, SAKURAI S. ET AL. 'Docosahexaenoyl-lysophosphatidylcholine as anticancer agent' page 77 ;colonne 2 ; cité dans la demande & JP,A,01 203 330 (...) 16 Aoüt 1989
- CHEMICAL ABSTRACTS, vol. 111, no. 21, 20 Novembre 1989, Columbus, Ohio, US; abstract no. 193395z, MIZUGUCHI T. ET AL. 'Antithrombotic and anticholesteremic nutrient compositions containing polyunsaturated fatty acids and lecithins' page 651 ;colonne 1 ; & JP,A,01 128 919 (...) 22 Mai 1989
- CHEMICAL ABSTRACTS, vol. 118, no. 13, 29 Mars 1993, Columbus, Ohio, US; abstract no. 116754h, HIBINO H. ET AL. 'Docosahexaenoates as LDL receptor activators for therapeutic uses' page 71 ; & JP,A,04 273 817 (...) 30 Septembre 1992
- LIPIDS vol. 25, no. 3 , 1990 pages 166 - 169 D.C. GAUDETTE ET AL. 'Albumin-found docosahexaenoic acid and collagen-induced human platelet reactivity'
- CHEMICAL ABSTRACTS, vol. 116, no. 15, 13 Avril 1992, Columbus, Ohio, US; abstract no. 150452b, IWAMURA S. ET AL. 'Antithrombotic powders containing highly unsaturated fatty acids, lecithins, and proteins (hydrolyzates) for foods' page 753 ; & JP,A,03 297 364 (...) 27 Décembre 1991

## Description

La présente invention a trait à de nouveaux médicaments à base d'acides gras polyinsaturés, et plus précisément à base d'acide docosahexaènoïque (encore appelé DHA ou 22:6n-3).

On sait que les tissus nerveux sont très riches en acides gras polyinsaturés essentiels (1, 2), notamment en DHA qui peut représenter 24 % des acides gras des phosphatidyléthanolamines (PE) de la matière grise (3). Les acides gras polyinsaturés jouent un rôle primordial dans le développement cérébral normal (4). Ainsi, la carence alimentaire en acides gras insaturés essentiels ou la perturbation de leur métabolisme pendant la période de développement cérébral, affecterait la myélinisation chez l'Homme (1, 5). Pour ce qui concerne plus particulièrement les acides gras de la famille n-3, il a été montré qu'une carence alimentaire en ces acides gras entraîne un développement pré- et post-natal incorrect de la rétine et du cerveau chez le singe Rhésus (6, 7) et d'autres animaux (8). Il semble en outre que ces acides gras soient impliqués dans la capacité d'apprentissage des jeunes rats (9, 1O). Le cerveau n'accumule pas le 18:3n-3, précurseur du 22:6n-3 ou DHA , d'une part parce qu'il possède les enzymes d'élongation et de désaturation nécessaires à la transformation du 18:3n-3 en 22:6n-3 (11) et d'autre part, parce qu'il peut capter le 22:6n-3 fixé sur l'albumine plasmatique fabriquée par le foie (12, 13) car le cerveau capte d'autant mieux les acides gras non estérifiés qu'ils sont insaturés (14, 15).

Sur la base des plus anciens de ces travaux, on a déjà proposé d'apporter à l'organisme des acides gras polyinsaturés essentiels, sous forme de nutriments ou de préparations administrables par d'autres voies. En particulier, plusieurs documents ont insisté sur l'intérêt d'apporter de cette façon des acides polyinsaturés essentiels tels que le 18:3n-3, l'acide arachidonique et le 22:6n-3. Dans les préparations envisagées, ces acides gras sont présents sous des formes diverses, en général mélangés et notamment sous forme de triglycérides, de phospholipides et également sous forme non estérifiée. Certains de ces documents soulignent l'intérêt d'administrer ces acides gras essentiels sous forme de composants des phospholipides.

Il a par ailleurs été montré que les régimes riches en acide eicosapentaènoïque (2O:5n-3) et en acide 22:6n-3 diminuent l'incidence des maladies cardiovasculaires (16). Les mécanismes responsables des effets bénéfiques de ces acides gras restent cependant à préciser. De nombreuses études se sont focalisées sur les effets de ces acides gras polyinsaturés sur les fonctions plaquettaires. Il a été montré que ces acides gras, comme d'autres acides gras cis-insaturés, inhibent l'agrégation des plaquettes induite par une grande variété d'activateurs plaquettaires dont l'U 46,619, agoniste du récepteur du thromboxane (TXA₂) et de la prostaglandine H₂ (PGH₂) (17-23). Les effets inhibiteurs du 2O:5n-3 et du 22:6n-3 s'exerceraient à plusieurs niveaux (17-19, 24-27). D'autre part, il a été montré que les 2O:5n-3 et 22:6n-3 non estérifiés inhibent de façon compétitive l'agrégation plaquettaire induite par l'U 46,619 ainsi que la liaison spécifique de ce dernier aux plaquettes lavées (28). L'incorporation in vitro du 2O:5n-3 et du 22:6n-3 dans les réserves de lipides plaquettaires, grâce à l'albumine, entraîne également la perte d'agrégabilité des plaquettes en réponse à l'U 46,619 et la diminution de l'affinité de l'agoniste pour son récepteur (29). Dans cette étude, si le 2O:5n-3 et le 22:6n-3 ont été estérifiés majoritairement dans les phospholipides, les effets observés peuvent aussi être attribués à l'enrichissement des autres fractions lipidiques en ces acides gras. Le 2O:5n-3 et le 22:6n-3 présentent une certaine sélectivité d'action sur le récepteur TXA₂/PGH₂ puisque aucun de ces acides gras n'affecte l'agrégation de plaquettes induite par la thrombine et par le ionophore A 23187, utilisés aux concentrations ne faisant pas intervenir ce récepteur (29). De plus, ils ne modifient pas la liaison de la yohimbine au récepteur alpha 2 adrénergique des membranes plaquettaires (30) qui présente une certaine homologie de structure. Parmi l'ensemble des acides gras testés, seul le 2O:5n-3 et le 22:6n-3 altèrent de façon spécifique le récepteur TXA₂/PGH₂ des plaquettes entières (29) ou des membranes plaquettaires solubilisées (30). Le 22:6n-3 semble présenter une efficacité supérieure à celle du 2O:5n-3.

L'utilisation de phosphatidylcholines "naturelles" obtenues à partir de poissons contenant de l'acide docosahexaénoïque en position sn-2 (PCDHAn) comme anticholestérolémiques, antithrombotiques, anti-agrégants plaquettaires a été décrite dans la demande JP-A-64 50890 par Nishizawa et al. (31).

L'utilisation des PCDHAn comme agents anti-tumoraux a été décrite dans la demande JP-A-1 160 989 par Hibino et al. (32).

D'autre part, l'utilisation du DHA-lysophosphatidylcholine en position sn-1 comme agent antitumoral a été décrite par Sakurai et al, dans la demande JP-A-1 203 330 (33).

La présente invention se propose de fournir de nouveaux médicaments à base d'acides gras polyinsaturés essentiels présentant une très grande efficacité. L'un des objectifs est ainsi de réaliser un nouveau médicament à effet anti-thromboxane A₂ extrêmement efficace.

Un autre objectif de l'invention est de réaliser un nouveau médicament permettant d'amener, dans le cerveau, un acide gras essentiel avec une efficacité particulièrement élevée.

L'invention a permis de découvrir, de façon surprenante, que l'efficacité, dans les applications précitées, du 22:6n-3 ou DHA doit être attribuée à son estérification dans les phosphatidylcholines (PC) à l'exclusion des autres formes de phospholipides et notamment des phosphatidyléthanolamines (PE), et ceci aussi bien dans l'application en tant qu'anti-thromboxane A₂, qu'en tant que source d'acide gras polyinsaturé essentiel apporté au cerveau. De plus, l'efficacité du DHA sous cette forme est particulièrement élevée par rapport à l'efficacité d'autres acides gras essentiels tels que l'acide linoléique (18:2n-6), y compris sous forme de phosphatidylcholine (PC).

L'administration per os de phosphatidylcholines naturelles contenant des acides gras à chaînes longues se heurte cependant à la lenteur de leur digestion. Or, dans de nombreux cas, l'administration par voie intra-veineuse n'est pas souhaitable.

L'invention a donc pour but important, celui de fournir des médicaments répondant aux objectifs précités qui puissent être administrés de façon très efficace, par voie orale, et qui, de plus, soient distribués avec une efficacité accrue, aux cellules réceptrices.

L'invention a pour objet de nouveaux médicaments à base d'acides gras essentiels, caractérisés en ce qu'ils comportent, en quantité thérapeutiquement efficace, au moins un composé choisi dans le groupe formé par :
- le DHA estérifié sous forme de lysophosphatidylcholine (lyso-PCDHA) en position sn-2;
- les DHA-phosphatidylcholines dans lesquelles le DHA est estérifié en position sn-2 et qui présentent un groupe acyle de très faible longueur en position sn-1 ; ces composés sont appelés ici PCDHAs ;
- et les triglycérides dans lesquels le DHA est estérifié en position sn-2 et qui présentent des groupes acyles de très faible longueur en positions sn-1 et sn-3.

Par groupe acyle de très faible longueur, on entend un groupe acyle pouvant comporter de 2 à 6 atomes de carbone, principalement l'acétyle, et éventuellement le propionyle et le butyryle.

Les composés utilisés dans les médicaments selon l'invention sont acylés en position sn-1 (et sn-3 dans le cas des triglycérides) par synthèse.

De préférence, les médicaments comportent, au moins 7O % de DHA dans la classe des acides gras estérifiés en position sn-2 des phosphatidylcholines, ou des triglycérides en quantité thérapeutiquement efficace, et de préférence plus de 10 %.

Dans une forme de réalisation préférée, la composition médicamenteuse peut être pure, c'est-à-dire ne comporter pratiquement que le ou les composés précités en tant que source d'acide gras essentiel polyinsaturé.

Dans une forme de réalisation avantageuse pour la fixation intracérébrale, le médicament contient, à titre de principe actif, le DHA estérifié sous forme de lysophosphatidylcholine (lyso-PCDHA) avec, de préférence, au moins 7O % de DHA dans la classe des acides gras essentiels polyinsaturés estérifiés en position sn-2 des lyso-PCDHA, et particulièrement supérieure à 1O %.

Chaque dose de médicament selon l'invention comporte une quantité thérapeutiquement efficace de PCDHAs ou de lyso-PCDHA.

L'invention a également pour objet l'utilisation des composés précités pour la préparation d'un médicament à effet anti-agrégant plaquettaire, utilisable notamment pour le traitement préventif ou curatif des maladies cardiovasculaires, y compris de la maladie athéromateuse.

L'invention a également pour objet l'utilisation des composés précités pour la préparation d'un médicament destiné au traitement des insuffisances ou des carences cérébrales en acides gras essentiels, notamment chez le prématuré, le nourrisson, le dénutri et le vieillard.

Les médicaments selon l'invention peuvent être préparés pour une administration orale ou parentérale et notamment intraveineuse, ou par voie rectale ou toute autre vois d'administration et notamment sous forme de collyre à usage ophtalmique.

Les préparations à administration orale peuvent comporter tout excipient ou véhicule usuel, par exemple ceux déjà utilisés pour l'administration d'acides gras essentiels. Elles peuvent consister en poudres, granulés, solutions ou autres et, éventuellement, incorporer d'autres principes actifs.

Pour une administration parentérale, on préfère réaliser le médicament sous forme d'un soluté de perfusion ayant la composition habituelle de ces solutés et dans lequel la concentration de PCDHAs (de préférence sous forme de liposomes ou liés à l'albumine) ou de lyso-PCDHA (lié à l'albumine) ou de triglycérides-DHA est de préférence de l'ordre de 1 à 1OO mg/litre de l'une ou l'autre des formes lipidiques de DHA.

L'administration orale peut être ponctuelle, par cure de plusieurs jours ou plusieurs semaines ou être chronique. La posologie est de préférence de l'ordre de 1 à 100 mg par kg par jour.

Les PCDHAs et la lyso-PCDHA peuvent être obtenues par synthèse chimique et/ou par biosynthèse, notamment à partir de produits de départ (acides gras, phospholipides, triglycérides) extraits et purifiés à partir de sources usuelles comme le placenta, les algues, les oeufs, les poissons, les organes animaux et les végétaux comme le soja.

Les PCDHAs peuvent passer plus facilement la barrière intestinale sans être dégradées. Si elles sont hydrolysées par des activités lipases agissant aussi bien en position sn-1 qu'en position sn-2, les acides acétique, propionique, butyrique et caproïque seront préférentiellement hydrolysés par rapport aux acides gras à chaîne longue en position sn-2 et, plus particulièrement, par rapport au DHA, connu pour être difficilement hydrolysable par les lipases humaines. Par exemple, si les PCDHAs sont hydrolysées par la lipase pancréatique, elles passeront exclusivement ou presque la barrière intestinale sous la forme de 1-lyso-2-DHA-glycérophosphocholine (désignée ci-après lyso-PCDHA) dont la réestérification en PCDHAn est favorisée dans les entérocytes. Les PCDHAs sont aussi facilement hydrolysables par des lipases spécifiques de la position sn-1. Il en résulte qu'une proportion nulle ou faible du DHA des PCDHAs sera sous forme non estérifiée après l'administration des PCDHAs. Par conséquent, comme la transformation du DHA en acide eicosapentaènoïque (EPA) est connue pour se faire avec l'acide gras non estérifié, elle peut être totalement ou presque supprimée *in vivo*. La formation d'EPA à partir des PCDHA administrées doit être évitée car l'EPA a des activités distinctes du DHA (Von Schacky et Weber, 1985 (34) ; Triggiani et coll.., 1990 (35) ; Robinson, 1993 (36) ; Salem et Ward, 1993 (37)) et contribue à réduire l'activité du DHA dans certains cas (Carlson et Coll., 1992 (38) ; Carlson, 1993 (39)).

Après l'administration des PCDHAs (intraveineuse ou per os), une proportion significative des PCDHAs est transformée en lysoPCDHA qui peut être réacylée avec des acides gras à chaîne longue pour donner des PC équivalents aux PCDHAn ou s'associer à des transporteurs comme l'albumine. En étant intactes, les PCDHAs par rapport aux PCDHAn présentent également l'avantage de mieux se fixer *in vitro* sur l'albumine ou d'être mieux transportées *in vivo* par l'albumine. Par comparaison aux PCDHAn, les PCDHAs peuvent ainsi avoir un devenir plus proche de celui de la lyso-PCDHA qui a été décrite ci-dessous comme une forme priviligiée d'apport du DHA au cerveau. L'albumine est connue également pour transporter de façon efficace des acides gras ou sous forme de lysophospholipides aux cellules, notamment les plaquettes.

Par contre, les PCDHAs comme les PCDHAn peuvent s'associer aux différentes lipoprotéines comme les HDL. Par conséquent, elle présentent également un métabolisme proche de celui des PCDHAn. Elles peuvent ainsi réduire la concentration plasmatique du cholestérol pro-athérogène associé aux LDL, en facilitant le transfert du cholestérol des LDL vers les HDL comme cela a été observé avec des PC contenant des acides gras à chaîne longue dont l'un d'entre eux au moins est polyinsaturé (Kirsten et coll., 1989 (40) ; O'Brien et coll., 1993 (41)). Cette-propriété des PCDHAs peut ainsi contribuer à diminuer l'incidence des maladies cardio-vasculaires.

Les PCDHAs présentent donc des propriétés originales par rapport aux PCDHAn préparées à partir de poissons (Hibino et al. (32) ; Nishizawa et al. (31)) et qui sont connues pour ne pas avoir en position sn-1 des acides gras à chaîne courte (acides gras comportant jusqu'à 6 atomes de carbone) (Sargent et al., 1990 (42)). En plus de leurs activités propres, elles peuvent se comporter à la fois comme des lysoPCDHA et des PCDHAn. Notamment, comme les PCDHAn, elles présentent l'avantage de pouvoir être administrables sous forme de liposomes, en principe, par toutes les voies disponibles y compris la voie intraveineuse, connue pour faciliter l'apport de drogues au cerveau. Dans une certaine mesure, elles constituent également une forme galénique stable des lyso-PCDHA. En effet, les PCDHAs sont partiellement hydrolysées en lyso-PCDHA *in vivo*. D'autre part, l'acylation de la position sn-1 des lyso-PCDHA évite la formation de 1-DHA-2-lyso-glycérophosphocholine au cours du stockage des lyso-PCDHA avant leur administration, voire même après leur administration. La 1-DHA-2-lyso-glycérophosphocholine est un phospholipide dont l'utilisation comme agent anti-tumoral a été décrite (Sakurai et al. (33)). Sa formation doit être évitée car ce composé a un métabolisme potentiellement différent des lyso-PCDHA (Morash et coll., 1989 (43)).

D'autres avantages et caractéristiques de l'invention apparaîtront à la lecture de la description suivante, faite à titre d'exemple non limitatif et se référant au dessin annexé dans lequel :

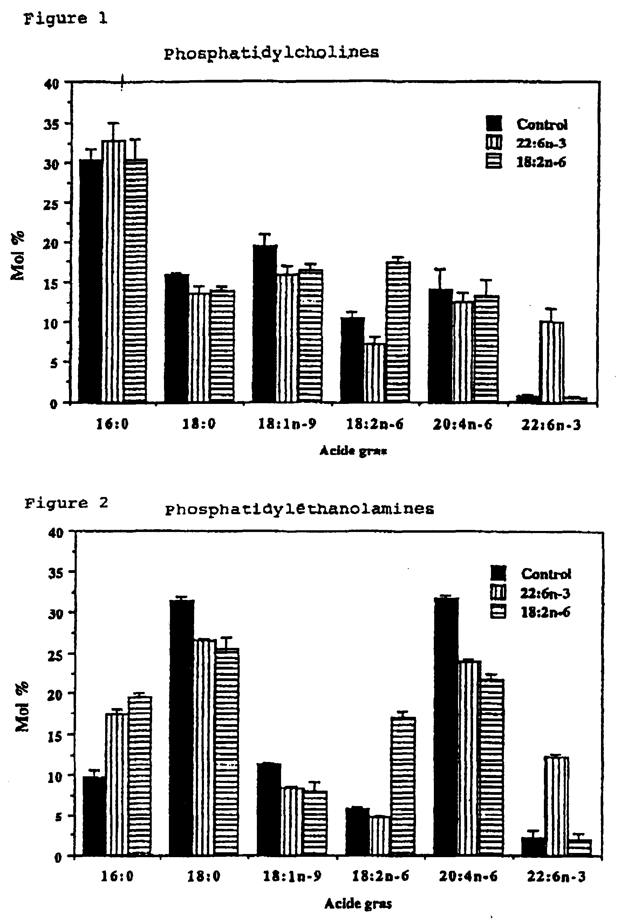

la figure 1 représente la composition en acides gras des diacyl-glycérophosphocholines des membranes plaquettaires après transfert de 1-palmitoyl-2-docosahexaènoyl-glycérophosphocholine (PC-22:6) et de 1-palmitoyl-2-linoléoyl-glycérophosphocholine (PC-18:2).

Les diacyl-glycérophosphocholines des membranes plaquettaires ont été remplacées soit par la 1-palmitoyl-2-docosahexaènoyl-glycérophosphocholine (barres à rayures verticales, "22:6n-3"), soit par la 1-palmitoyl-2-linoléoyl-glycérophosphocholine (barres à rayures horizontales, "18:2n-6"), en utilisant la protéine de transfert endogène des plaquettes, spécifique des phosphatidylinositols et des phosphatidylcholines. Les membranes plaquettaires témoin (barres noires, "control") sont obtenues par incubation sans addition de phospholipides. Les données du graphique représentent la moyenne ± S.D. de trois essais indépendants.

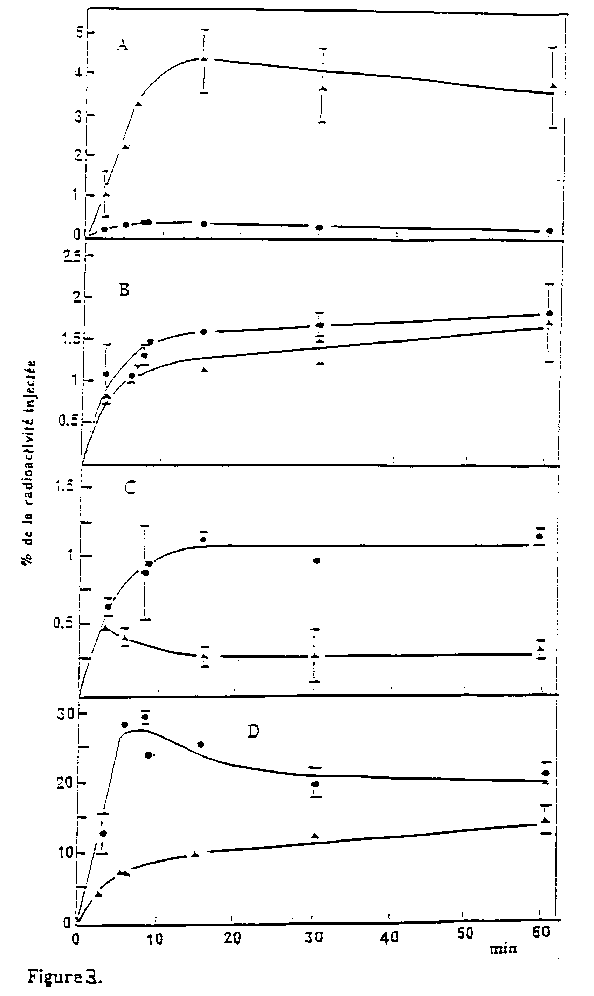

la figure 2 représente la composition en acides gras des diacyl-glycérophosphoéthanolamines des membranes plaquettaires après transfert de 1-palmitoyl-2-docosahexaènoyl-glycérophosphoéthanolamine (PE-22:6 et de 1-palmitoyl-2-linoléoyl glycérophospho-éthanolamine (PE-18:2).

Les diacyl-glycérophosphoéthanolamines des membranes plaquettaires ont été remplacées soit par la 1-palmitoyl-2-docosahexaènoyl-glycérophosphoéthanolamine (barres à rayures verticales, "22:6n-3"), soit par la 1-palmitoyl-2-linoléoyl-glycérophosphoéthanolamine (barres à rayures horizontales "18:2n-6"), en utilisant la protéine de transfert de lipides purifiée à partir de maïs. Les membranes plaquettaires témoin (barres noires, "control") sont obtenues par incubation sans addition de phospholipides. Les données du graphique représentent la moyenne ± S.D. de trois essais indépendants.

la figure 3 représente l'évolution de la radioactivité recouvrée dans les lipides totaux : A du cerveau, B du rein, C du coeur et D du foie, après injection de DHA tritié sous forme libre (cercle noir) ou sous forme de 2-DHA-1-lyso PC (triangle noir).

### Exemple 1 : Préparation d'une composition purifiée en lyso-PCDHA et en PCDHAs à partir de phosphatidylcholines (PC) d'algues.

### a) Culture de microalgues produisant du PCDHA

Une dinoflagellée hétérotrophe, Crypthecodinium cohnii, est cultivée en milieu AXM à 25°C. Les cultures sont aérées et agitées dans les conditions décrites par BEACH et HOLZ (44). Après 4 jours de culture, les cellules sont récupérées par centrifugation et la biomasse humide est lyophilisée. Un fermenteur contenant 1O litres de milieu permet d'obtenir dans ces conditions environ 3g de biomasse lyophilisée.

### b) Extraction des lipides

Dans un premier temps, les lipides neutres sont extraits de la biomasse lyophilisée avec de l'hexane selon le procédé utilisé classiquement pour les huiles végétales.

Dans un second temps, la biomasse est reprise par un solvant alcoolique (méthanol ou éthanol) pour l'extraction des lipides polaires et en particulier des phospholipides selon le protocole décrit dans le brevet européen n° O 298 787.

Ce procédé permet d'extraire environ 18 % de lipides neutres et 12 % de lipides polaires (teneurs exprimées en poids et rapportées à la biomasse lyophilisée).

### c) Purification des phosphatidylcholines contenant le DHA en position sn-2.

La phosphatidylcholine contenant le DHA est isolée des lipides polaires par chromatographie liquide haute performance (HPLC) selon le protocole décrit par CHRISTIE et al. (45). Le traitement de la totalité des lipides polaires obtenus à partir de 1O litres de culture (environ 36O mg) permet de purifier environ 18O mg de PCDHA. La phosphatidylcholine obtenue contient 66 % de DHA (teneur exprimée en poids des acides gras totaux estérifiés sur la PC).

### d) Préparation des lysoPCDHA.

Les chaînes acyles en position sn-1 des phosphatidylcholines purifiées comme indiqué ci-dessus sont hydrolysées par toute lipase ayant une activité phospholipase A₁. La lysoPCDHA obtenue est purifiée par chromatographie.

### e) Préparation des PCDHAs.

La lyso-PCDHA décrite ci-dessus peut être réacylée par synthèse organique pour obtenir les PC-DHA acylées avec les acides acétique, propionique, butyrique ou caproïque en position sn-1 (Delfino et coll., 1987 (46)). Les PCDHAs obtenues sont ensuite purifiées par chromatographie.

### Exemple 2 : Préparation d'une composition purifiée en PC-DHAs par semi-synthèse à partir de glycérophosphorylcholine ou de glycérophosphate.

a) Diacylation de la glycérophosphocholine et du glycérophosphate avec les acides acétique, propionique, butyrique, caproïque ou docosahexaènoïque.
   Le glycérophosphate est acylé avec des dérivés des acides acétique, propionique, butyrique, caproïque ou docosahexaènoïque (anhydride d'acyle, chlorure d'acyle, imidazolide d'acyle ...) pour donner un acide diacylphosphatidique (PA). Le PA formé est ensuite converti en PC en utilisant le chlorure de choline (Schena et Davis, 1989 (47); Walts et coll., 1992 (48)).
   Les PC peuvent être obtenues plus directement en acylant la glycérophosphorylcholine avec les dérivés des acides acétique, propionique, butyrique, caproïque ou docosahexaènoïque comme décrit ci-dessus.
b) Préparation des PCDHAs à partir des PC contenant les résidus acétique, propionique, butyrique ou caproïque en position sn-1 et sn-2.
   Les PC contenant les résidus acétique, propionique, butyrique ou caproïque sont sélectivement hydrolysées en position sn-2 par toute lipase ayant une activité phospholipase A₂. Les lysoPC formées sont réacylées avec des dérivés du DHA (anhydride d'acyle, chlorure d'acyle, imidazolide d'acyle ...) pour donner des PCDHAs.
c) Préparation des PCDHAs à partir des PC contenant le résidu docosahexaènoïque en position sn-1 et sn-2.
   La PC contenant le résidu DHA est sélectivement hydrolysée en position sn-1 par toute lipase ayant une activité phospholipase A₁. Les lysoPC formées sont réacylées avec des dérivés des acides acétique, propionique, butyrique ou caproïque (de préférence anhydride d'acyle ou chlorure d'acyle) pour donner des PCDHAs

### Exemple 3 : Etude de l'effet anti-thromboxane A₂ des 1-acyl-2-docosahexaènoyl-glycérophosphocholines.

On a réalisé des préparations de DHA estérifié dans différentes classes de phospholipides en utilisant des protéines de transfert de phospholipides pour enrichir de manière spécifique, les phosphatidylcholines (PC) et les phosphatidyléthanolamines (PE) des membranes plaquettaires en acide linoléique (18:2n-6) et en DHA (49). Notamment la protéine de transfert (PT), spécifique des PC, purifiée à partir de foie de boeuf, a été employée pour modifier la composition en espèces moléculaires des PC des érythrocytes (49).

Dans le procédé utilisé dans le présent exemple, la quantité endogène de phospholipides plaquettaires n'est pas altérée par le transfert effectué à l'aide de la PT de maïs ou de plaquettes ; cependant, les phospholipides ont été enrichis en 18:2n-6 ou en DHA.

L'influence des acides gras sur le récepteur TXA₂/PGH₂ a été établie en mesurant la liaison spécifique du SQ29,548 tritié, un antagoniste compétitif du récepteur TXA₂/PGH₂, à des membranes plaquettaires dont les PC ou les PE sont enrichies soit en 18:2n-6 soit en DHA.

Seules les membranes plaquettaires contenant des PC enrichies en DHA altèrent le récepteur TXA₂/PGH₂ en augmentant de façon significative la constante de dissociation du SQ29,548. L'enrichissement des PC des membranes plaquettaires en 18:2n-6 et des PE en 18:2n-6 ou en DHA n'a pas d'effet sur le récepteur (Tableau I).

Ces propriétés font considérer les PCDHAs, à une concentration thérapeutiquement active, comme un médicament ayant une activité anti-agrégante et anti-hypertensive par son effet anti-thromboxane A₂.

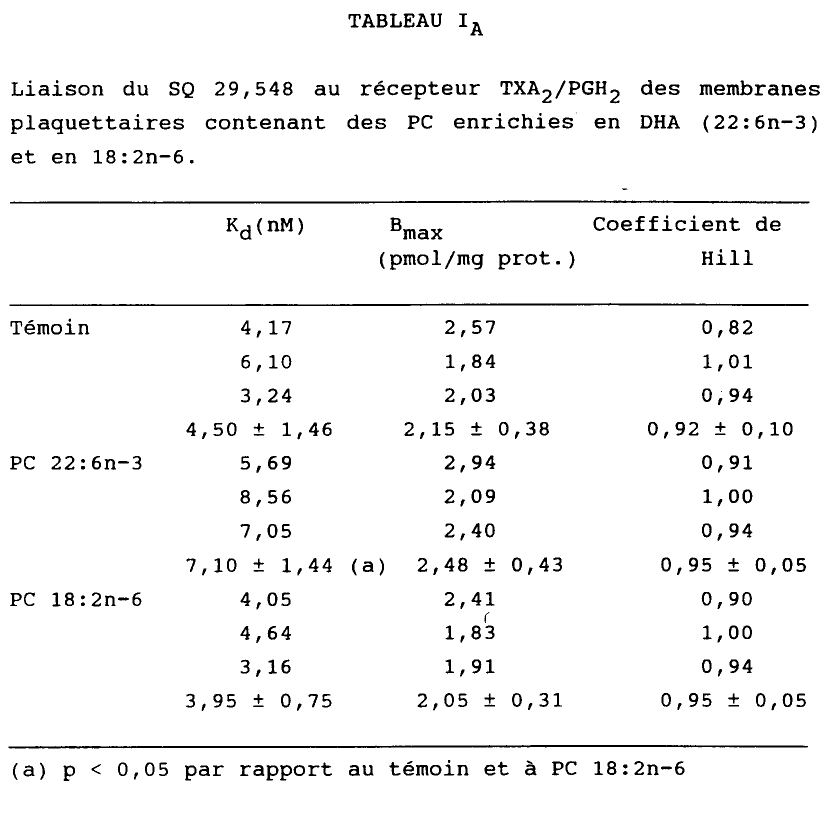

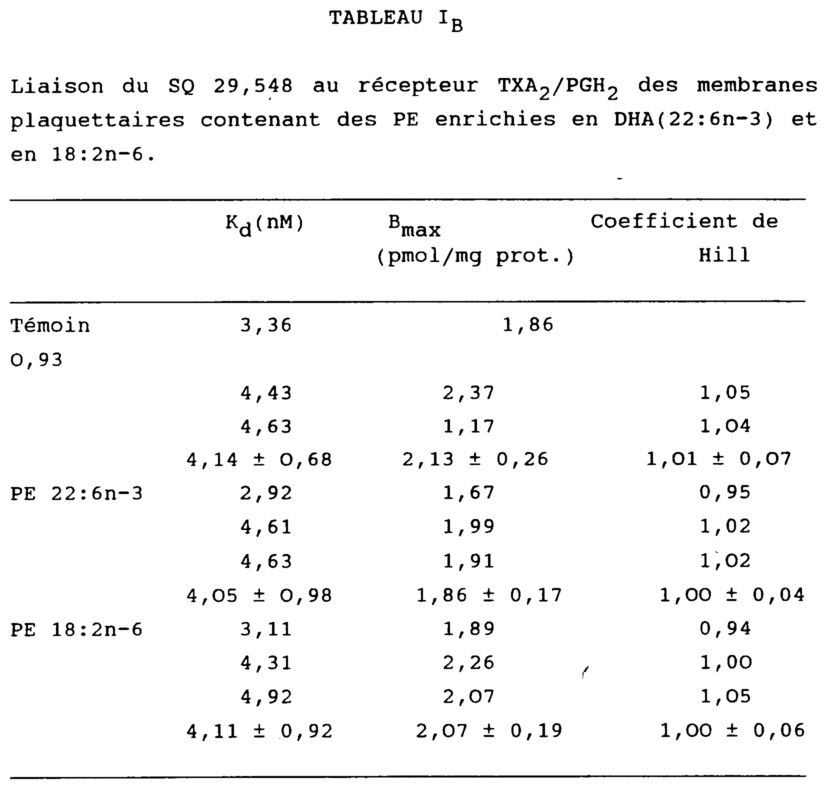

### Exemple 4 : Etude de la captation par le cerveau chez le rat, des 2-acyl, 1-lysophosphatidylcholines (lyso-PC).

L'animal étudié est le rat de 2O jours chez lequel le métabolisme lipidique cérébral est à son apogée.

Les lyso PC sont marqués sur l'acide gras et la choline, fixés sur albumine et administrés par voie intraveineuse (50). Les résultats montrent- que, pour le DHA(22:6n-3), les lyso-PC le renfermant sont particulièrement bien captés par le cerveau et cela mieux que les lyso-PC comprenant d'autres acides gras tels que 18:1, 18:2 et 2O:4. Seul le cerveau montre une telle préférence comme on le voit sur la figure 3, puisque à l'exception du rein, qui capte sensiblement de la même façon les deux formes d'apport, à savoir le lyso-PCDHA et le DHA sous forme non estérifiée, le foie et le coeur captent moins bien les lyso-PCDHA que le DHA non estérifié. Il apparaît que la réacylation du DHA dans les phosphatidylcholines est faible et n'est visible que dans les premiers temps après l'injection (tableau II) car très rapidement il se forme une estérification différentielle dans les phosphatidyl éthanolamines. Il apparaît donc que les lyso-PCDHA fixés sur albumine constituent une forme d'apport de DHA particulièrement préférentielle au cerveau en développement.

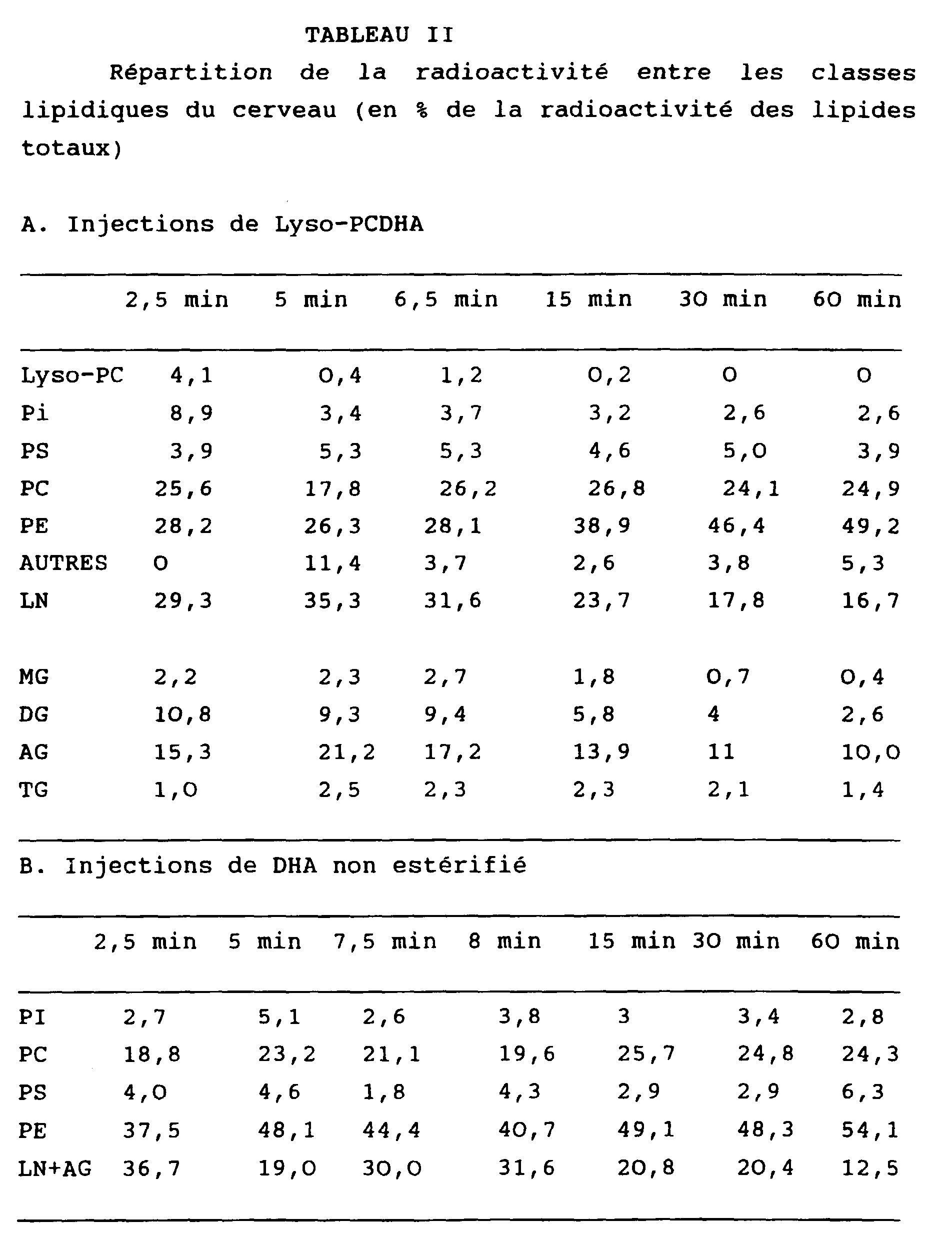

### Bibliographie

1. Bourre J.M. (198O) INSERM symposium 14, (N. Baumann ed.), Elsevier, North Holland Biomedical Press, 187-2O6.
2. Martinez M. et Ballabriga A. (1987) Lipids, 22, 133-138.
3. O'Brien J.S. et Sampson E.L. (1965) J. Lipid Res. 6,545-547.
4. Alling C., Bruce A., Karlsson L., Sapia O. et Svennerholm L. (1972) J. Nutr. 1O2, 773-782.
5. Gozzo S. et D'Udine B. (1977) Neurosci. Lett. 7,267-275.
6. Neuringer M., Connor W.E., Lin D.S., Barstad L. et Luck S. (1987) Proc. Natl. Acad. Sci. U.S.A. 83, 4O21-4O25.
7. Connor W.E., Neuringer E.M., Barstad L. et Lin D.S. (1984) Trans. Assoc. Am. Physicians 97, 1-19.
8. Salem N. Jr., Kim H.Y. et Yergey J.A. (1986) Simopoulos A.P., Kifer R.R., et Martin R. (eds.) Academic Press, New-York, 263-317.
9. Yamamoto N., Saitoh M., Moriuchi A., Nomura M. et Okuyama H. (1987) J. Lipid Res. 28, 144-151.
1O. Bourre J.M., François M., Youyou A., Dumont O., Piciotti M., Pascal G. et Durand G. (1989) J. Neurochem. 43, 342-348.
11. Cook H.W. et Spence M.W. (1974) Biochim. Biophys. Acta 369, 129-141.
12. Burton L. Scott M. et Bazan N.G. (1989) Proc. Natl. Acad. Sci. U.S.A. 86, 29O3-29O7.
13. Li J., Wetzel G. et O'Brien (1992) J. Lipid Res. 33, 539-548.
14. Hassan A.G. et Crawford M.A. (1976) J. Neurochem. 27, 967-968.
15. Anderson G.J. et Connor W.E. (1988) Lipids 23, 286-29O.
16. Leaf, A. et Weber, P.C. (1988) New Engl. J. Med. 318, 549-557.
17. MacIntyre, D.E., Hoover, R.L., Smith, M., Steer, M., Lynch, C., Karnosky, M.J. et Salzman, E.W. (1984) Blood 63, 848-857.
18. Kitagawa, S., Endo, J. et Kametami, F. (1985) Biochim. Biophys. Acta 818, 391-397.
19. Sato, T., Hashizume, T., Nakao, K. Akiba, S. et Fujii, T. (1989) Biochim. Biophys. Acta 992, 168-173.
2O. Sato, T., Nakao, K., Hashizume, T. et Fujii, T. (1987) Biochim. Biophys. Acta 931, 157-164.
21. Gryglewski, R.J., Salmon, J.A., Ubatuba, F.B., Weatherly, B.C., Moncada, S. et Vane, J.R. (1979) Prostaglandins 18,453-478.
22. Croset, M. et Lagarde, M. (1986) Thromb. Haemostas. 56, 57-62.
23. Hatmi, M., Lussiana, J.P. Junien, J.L., Bure, J. et Vargaftig, B.B. (1988) Biochem. Pharmacol. 37, 481-489.
24. Ballou, L.R. et Cheung. W.Y. (1985) Proc. Natl. Acad. Sci. U.S.A. 82, 371-375.
25. Needleman, S.W., Spector, A.A. et Hoak, J.C. (1982) Prostaglandins 24, 6O7-622.
26. Corey, E.J., Shih, C. et Cashman, J.R. (1983) Proc. Natl. Acad. Sci. USA 8O, 3581-3584.
27. Abeywardena, M.Y., McLennan, P.L. et Charnock, J.S. (1991) Am. J. Physiol. 26O, H379-H385.
28. Swann, P.G. Venton, D.L. et Le Breton, G.C. (1989) FEBS Lett. 243, 244-246.
29. Swann, P.G., Parent, C.A. Croset, M., Fonlupt, P., Lagarde, M., Venton, D.L. et Le Breton, G.C. (199O) J. Biol. Chem. 243, 244-246.
3O. Parent, C.A. Lagarde, M. Venton, D.L. et Le Breton G.C. (1992) J. Biol. Chem. 267, 6541-6547.
31. Nishizawa Y., Isoda Y., Kageyama H. et Sakai K., Nippon Oil and Fat Co. et Yaizu Suisan Kagaku Industry Co., Jpn. Kokai Tokkyo Koho, JP-A-64 50 890, 1989, déposée le 20 août 1987.
32. Hibino H., Fukuda N., Nakachi O., Sakurai S., Asahi K. et Takahashi N., Nippon Oil and Fat Co. et Institute of Physical and Chemical Research, Jpn. Kokai Tokkyo Koho, JP-A-01 160 989, 1989, déposée le 18 décembre 1987.
33. Sakurai S., Asahi K., Takahashi N., Hibino H. et Fukuda N., Institute of Physical and Chemical Research et Nippon Oil and Fat Co., Jpn. Kokai Tokkyo Koho, JP-A-01 203 330, 1989, déposée le 5 février 1987.
34. Von Schacky C. et Weber P.C., J. Clin. Invest., 1985, Vol. 76, p. 2446-2450.
35. Triggiani M., Connell T.R. et Chilton F.H., J. Immunol., 1990, Vol. 145, p. 2241-2248.
36. Robinson D.R., Xu L.L., Tateno S., Guo M. et Colvin R.B., J. Lipid Res., 1993, Vol. 34, p. 1435-1444.
37. Salem N. et Ward G.R. dans: Nutrition and fitness in health and disease, World Rev. Nutr. Diet. (Ed. A.P. Simopoulos), 1993, Vol. 72, pp. 127-147. Basel: S. Karger.
38. Carlson S.E., Cooke R.J., Werkman S.H. et Tolley E.A., Lipids, 1992, Vol. 27, p. 901-907.
39. Carlson S.E. dans: Lipids, learning and the brain: fats in infant formulas, Ed. J. Dobbing, 1993, pp. 188-207. Columbus, OH: Ross Laboratories.
40. Kirsten R., Heintz B., Nelson K. et Oremek G., Int. J. Clin. Pharmacol. Ther. Toxicol., 1989, Vol. 27, p. 129-134.
41. O'Brien B.C. et Andrews V.G., Lipids, 1993, Vol. 28, p. 7-12.
42. Sargent J.R., Bell M.V., Henderson R.J. et Tocher D.R. dans: Animal Nutrition and Transport Processes. 1. Nutrition in Wild and Domestic Animals Comp. Physiol. Ed. J. Mellinger, 1990, Vol. 5, pp. 11-23.
43. Morash S.C., Cook H.W. et Spence M.W., Biochim. Biophys. Acta, 1989, Vol. 1004, p. 221-229.
44. Beach, D.H., et Holz. Jr, G.G., (1973) Biochim. Biophys. Acta 316, 56-65.
45. Christie, W.W., J. Lipid Res. (1985) 26, 5O7-512.
46. Delfino J.M., Schreiber S.L. et Richard F.M., Tetrahedron Lett., 1987, Vol. 28, p. 2327-2330.
47. Schena D.G. et Davis J. T., to Genzyme Corp., US Pat. Appl., USSN 577 351, 1989.
48. Walts A.E., Schena D.G., Fox E.M. Davis J.T. et Mischke M.R., dans: Chirality in industry. Ed. A.N. Collins et J. Crosby, 1992, pp. 223-235. New York: John Wiley and sons Ltd.
49. Rueckert, D.G. et Schmidt K., (199O) Chem. Phys. Lipids 56, 1-2O.
50. Thiès F., Delachambre M.C., Bentejac M., Lagarde M. et Lecerf J., (1992) J. Neurochem. 59, 111O-1116.

## Revendications

1. Médicaments à base d'acides gras essentiels, caractérisés on ce qu'ils comportent, en quantité thérapeutiquemont efficace, au moins un composé choisi dans le groupe formé par :
- le DHA estérifié sous forme de lysophosphatidylcholine (lyso-PCDHA) en position sn-2 ;
- les DHA-phosphatidylcholines (PCDHAs) dans lesquelles le DHA est estérifié on position sn-2 et qui présentent un groupe acyle de très faible longueur en position sn-1 ;
- et les triglycérides dans lesquels le DHA est estérifié on position sn-2 et qui présentent des groupes acyles comportant de 2 à 6 atomes de carbone en positions sn-1 et sn-3.

2. Médicaments selon la revendication 1, caractérisés en ce que le groupement acyle est l'acétyle, le propanoyle ou le butyryle.

3. Médicaments selon l'une des revendications 1 et 2, caractérisés on ce qu'ils comportent au moins 70 % d'acide docosahexaènoïque (DHA) dans la classe des acides gras estérifiés on position sn-2.

4. Médicaments selon l'une des revendications 1 et 2, caractérisés on ce qu'ils comportent, à titre de principe actif, au moins 1O % d'acide docosahexaènoïque (DHA) dans la classe des acides gras estérifiés on position sn-2 des lysophosphatidylcholines.

5. Utilisation des composés définis dans l'une des revendications 1 et 2 pour la préparation d'un médicament à effet anti-agrégant plaquettaire, notamment pour le traitement préventif ou curatif dos maladies cardiovasculaires.

6. Utilisation des composés définis dans l'une des revendications 1 et 2, pour la préparation d'un médicament pour le traitement des insuffisances ou carences cérébrales en acides gras essentiels.

7. Médicaments selon l'une quelconque des revendications 1 à 3, caractérisés en ce qu'il contient la quantité de principe actif nécessaire pour une posologie de 1 à 1OO mg/kg/jour.

8. Médicaments selon l'une quelconque des revendications 1 à 4 et 7, conditionnnés pour une administration par voie orale.

9. Procédé de préparation d'une composition enrichie ou purifiée en PCDHAs ou en lyso-PCDHA, dans lequel on extrait les phospholipides d'une biomasse par un solvant alcoolique, et on isole la phosphatidylcholine contenant le DHA par chromatographie liquide haute performance, puis on hydrolyse le DHA par toute lipase ayant une activité phospholipase A₁, le lyso-PCDHA obtenu étant purifié par chromatographie.

10. Procédé selon la revendication 9, caractérisé en ce que l'on réacyle la position sn-1 par un acide choisi dans le groupe constitué des acides acétique, propionique, butyrique et caproïque.

11. Procédé de préparation d'une composition enrichie ou purifiée en PCDHAs, caractérisé en ce que l'on procède à une diacylation de la glycérophosphocholine ou de glycérophosphate à l'aide d'acide acétique, propionique, butyrique ou caproïque ou de leurs dérivés, puis que l'on hydrolyse sélectivement les positions sn-2 par une phospholipase A2, après quoi on réacyle les lyso-PC formés avec un dérivé du DHA.

12. Procédé de préparation d'une composition enrichie ou purifiée en PCDHAs, caractérisé en ce que l'on procède à une diacylation de la glycérophosphocholine ou de glycérophosphate à l'aide d'acide DHA, que l'on hydrolyse sélectivement les positions sn-1 par une phospholipase A1, après quoi on réacyle les lyso-PCDHA formés à l'aide d'acide acétique, propionique, butyrique ou caproïque ou de l'un de leurs dérivés.

## Claims

1. Medicines based on essential fatty acids, characterized in that they comprise, in a therapeutically effective quantity, at least one compound chosen from the group formed by :
- DHA esterified in the sn-2 position, in the form of lysophosphatidylcholine (lyso-PCDHA);
- the DHA-phosphatidylcholines (PCDHAs) in which the DHA is esterified in the sn-2 position and which has an acyl group of very short length in the sn-1 position;
- and the triglycerides in which the DHA is esterified in the sn-2 position and which has acyl groups, comprising from 2 to 6 carbon atoms, in the sn-1 and sn-3 positions.

2. Medicines according to Claim 1, characterized in that the acyl group is an acetyl, propanoyl or butyryl group.

3. Medicines according to one of Claims 1 and 2, characterized in that they comprise at least 70% docosahexenoic acid (DHA) in the class of fatty acids esterified in the sn-2 position

4. Medicines according to one of Claims 1 and 2, characterized in that they comprise, as principal active ingredient, at least 10% docosahexenoic acid (DHA) in the class of fatty acids esterified in the sn-2 position of the lysophosphatidylcholines.

5. Use of the compounds defined in one of Claims 1 and 2 for the preparation of a medicine with an anti-aggregating effect on platelets, particularly for the preventative or curative treatment of cardio-vascular diseases.

6. Use of the compounds defined in one of Claims 1 and 2, for the preparation of a medicine for the treatment of insufficiencies or a cerebral absence of essential fatty acids.

7. Medicines according to any one of Claims 1 to 3, characterized in that it contains the quantity of active ingredient required for a posology of from 1 to 100 mg/kg/day.

8. Medicines according to any one of Claims 1 to 4 and 7, prepared for administration by the oral route.

9. Method of preparing a composition enriched in or purified with respect to PCDHAs or lyso-PCDHA, in which the phospholipids are extracted from a biomass by an alcoholic solvent, the phosphatidylcholine containing the DHA is isolated by high performance liquid chromatography and the DHA is hydrolyzed by any lipase having A₁ phospholipase activity, the lyso-PCDHA obtained being purified by chromatography.

10. Method according to Claim 9, characterized in that the sn-1 position is acylated again by an acid chosen from the group made up of acetic, propionic, butyric and caproic acids.

11. Method of preparing a composition enriched in or purified with respect to PCDHAs, characterized in that a diacylation is carried out on the glycerophosphocholine or glycerophosphate using acetic, propionic, butyric or caproic acid or their derivatives and then the sn-2 positions are selectively hydrolyzed by an A2 phospholipase, after which the Lyso-PC formed is again acylated with a DHA derivative.

12. Method of preparing a composition enriched in or purified with respect to PCDHAs, characterized in that a diacylation is carried out on the glycerophosphocholine or glycerophosphate using docosahexenoic acid (DHA) and then the sn-1 positions are selectively hydrolyzed by an A1 phospholipase, after which the Lyso-PCDHA formed is again acylated using acetic, propionic, butyric or caproic acid or one of their derivatives.

## Patentansprüche

1. Arzneimittel auf Basis von Essenzfettsäuren, dadurch gekennzeichnet, dass sie in therapeutisch wirksamer Menge mindestens eine in der folgenden Gruppe gewählte Verbindung beinhalten :
- esterifiziertes DHA in Form von Lysophosphatidylcholin (Lyso-PCDHA) in Position sn-2 ;
- DHA-phosphatidylcholine (PCDHAs), in denen das DHA in Position sn-2 esterifiziert ist, und die eine Acylgruppe von sehr geringer Länge in Position sn-1 aufweisen ;
- und Triglyceride, in denen das DHA in Position sn-2 esterifiziert ist, und die Acylgruppen mit 2 bis 6 Kohlenstoffatomen in den Positionen sn-1 und sn-3 aufweisen.

2. Arzneimittel nach Anspruch 1, dadurch gekennzeichnet, dass die Acylgruppe aus Acetyl, Propanoyl oder Butyryl besteht.

3. Arzneimittel nach einem der Ansprüche 1 und 2, dadurch gekennzeichnet, dass sie mindestens 70 % docosahexaenoisches Fett (DHA) in der Klasse der esterifizierten Fettsäuren in Positon sn-2 aufweisen.

4. Arzneimittel nach einem der Ansprüche 1 und 2, dadurch gekennzeichnet, dass sie als Aktivstoffe mindestens 10 % docosahexaenoische Säure (DHA) in der Klasse der esterifizierten Fettsäuren in Position sn-2 der Lysophosphatidylcholine aufweisen.

5. Verwendung der in einem der Ansprüche 1 und 2 definierten Verbindungen zur Zubereitung eines plättchenförmigen Arzneimittels mit entlastender Wirkung, insbesondere für die vorbeugende oder heilende Behandlung von Herzgefäss-Krankheiten.

6. Verwendung der in einem der Ansprüche 1 und 2 definierten Verbindung für die Zubereitung eines Arzneimittels für die Behandlung von zerebralen Mängeln an Essenfettsäuren.

7. Arzneimittel nach einem der Ansprüche 1 bis 3, dadurch gekennzeichnet, dass sie die Mengen von Aktivstoffen enthalten, die für eine Dosierung von 1 bis 100 mg/kg/Tag erforderlich sind.

8. Arzneimittel nach einem der Ansprüche 1 bis 4 und 7, abgepackt für eine Einnahme durch Verschlucken.

9. Zubereitungsverfahren einer mit PCDHAs oder Lyso-PCDHA bereicherten oder gereinigten Zusammensetzung, bei dem man einer Biomasse anhand eines alkoholischen Lösemittels die Phospholipide entzieht, man das DHA enthaltende Phosphatidylcholin durch flüssige Hochleistung-Chromatographie abtrennt, und dann das DHA mit einer Lipase hydrolysiert, die eine Phospholipase-Aktivität A₁ hat, wobei das Lyso-PCDHA durch Chromatographie gereinigt wird.

10. Verfahren nach Anspruch 9, dadurch gekennzeichnet, dass man die Position sn-1 mit einer Säure reacyliert, die in der aus Essig-, Propen-, Butter- und Capronsäure bestehenden Gruppe gewählt wird.

11. Zubereitungsverfahren einer mit PCDHAs bereicherten oder gereinigten Zusammensetzung, dadurch gekennzeichnet, dass man anhand von Essig-, Propen-, Butter- oder Capronsäure oder ihrer Derivate eine Diacylation des Glycerophosphocholins oder des Glycerophosphats durchführt, dann die Positionen sn-2 selektiv mit einer Phospholipase A2 hydrolysiert, und schliesslich die gebildeten Lyso-PC mit einem Derivat des DHA reacyliert.

12. Zubereitungsverfahren einer mit PCDHAs bereicherten oder gereinigten Zusammensetzung, dadurch gekennzeichnet, dass man anhand von DHA-Säure eine Diacylation des Glycerophosphocholins oder des Glycerophosphats durchführt, dass man die Positionen sn-1 durch eine Phospholipase A1 selektiv hydrolysiert und danach die gebildeten Lyso-PCDHA anhand von Essig-, Propen-, Butter- oder Capronsäure oder ihrer Derivate reacyliert.
